# FASCICULE DE BREVET EUROPEEN

(11) **EP 2 874 588 B1**
(45) Date de publication et mention de la délivrance du brevet: **23.11.2016**
(21) Numéro de dépôt: 13773366.3
(22) Date de dépôt: 22.07.2013
(51) Int. Cl.: A61F 13/532, A61F 13/535, A61F 13/00, A61F 13/02, A61F 13/511, A61F 13/53

(54) **ARTICLE DESTINE A VENIR EN CONTACT AVEC UN LIQUIDE, NOTAMMENT PANSEMENT**
ARTIKEL ZUR HERSTELLUNG EINES KONTAKTS MIT EINER FLÜSSIGKEIT, INSBESONDERE EINES VERBANDS
ITEM INTENDED TO COME INTO CONTACT WITH A LIQUID, IN PARTICULAR A BANDAGE

(30) Priorité: 23.07.2012 FR 1257108
(43) Date de publication de la demande: 27.05.2015
(73) Titulaire: Commissariat à l'Énergie Atomique et aux Énergies Alternatives, 75015 Paris (FR); URGO RECHERCHE INNOVATION ET DEVELOPPEMENT, 21300 Chenôve (FR)
(72) Inventeur: FOUILLET, Yves, F-38340 Voreppe (FR); MARSIQUET, Cyril, F-16270 Roumazieres Laubert (FR); REVOL-CAVALIER, Frédéric, F-38180 Seyssins (FR); PERNOT, Jean-Marc, F-21000 Dijon (FR); LECOMTE, Serge, F-21000 Dijon (FR); LAMOISE, Michel, F-21110 Bessey Les Citeaux (FR)
(74) Mandataire: Nony
(86) Numéro de dépôt international: PCT/IB2013/056010
(87) Numéro de publication internationale: WO 2014/016759

(56) Documents cités:
- EP-A1- 0 875 222
- EP-A1- 1 974 705
- US-A- 4 676 785

## Description

La présente invention concerne des articles destinés à venir par leur côté proximal en contact avec des liquides, en particulier des liquides sécrétés par la peau, la plaie et/ou les muqueuses, pour gérer la propagation et le stockage de ces liquides et, plus particulièrement mais non exclusivement, des pansements à appliquer sur une plaie.

La gestion de la propagation des liquides ainsi que leur stockage sont des problèmes complexes à résoudre pour lesquels les articles proposés dans le domaine des pansements et des produits d'hygiène ne fournissent pas aujourd'hui des solutions entièrement satisfaisantes.

Ces articles doivent en effet satisfaire à un cahier des charges qui comprend des propriétés antagonistes. Une première exigence est d'éloigner le plus loin et le plus rapidement possible les liquides afin d'éviter à cause de leur accumulation tout phénomène de macération ou d'irritation au niveau de la peau, la plaie ou les muqueuses. Les liquides ne doivent pas s'accumuler mais il est aussi préférable d'éviter leur migration latérale à partir du lieu de sécrétion pour diminuer l'augmentation de la zone humidifiée à des fins de garantir une meilleure hygiène et des raisons de confort de l'utilisateur.

Ceci est particulièrement important lors de l'utilisation d'un pansement. Dans ce cas il est important d'éviter que la peau située en bordure de la lésion, appelée peau périlésionnelle, qui est très fragile, soit humide car cela peut provoquer une altération de la peau, favorisant par exemple une infection et/ou irritation.

Ainsi, il est souhaitable que le pansement draine efficacement les liquides corporels sécrétés par la plaie et notamment empêche une migration latérale de ces liquides de leur lieu de sécrétion vers la périphérie de ce lieu. Un tel drainage permet de maintenir la périphérie de la plaie au sec, d'où une meilleure hygiène et des conditions de cicatrisation améliorées.

Une seconde exigence est de stocker ces liquides et d'éviter leur retour vers la peau, la plaie ou les muqueuses. Un stockage important permet d'augmenter la durée d'utilisation de l'article. Dans le cas d'un pansement cette durée d'utilisation est particulièrement importante car on diminue le risque d'altérer le processus de cicatrisation de la plaie en changeant ce dernier moins souvent. Mais l'optimisation des capacités de stockage conduit, en raison de l'accumulation des liquides, à des articles plus épais et plus lourds pour lesquels les risques de fuites, de détachement à cause du poids de l'article, par exemple à cause du positionnement vertical du pansement sur un ulcère de jambe, et de retour des liquides de la zone de stockage vers la peau, la plaie ou les muqueuse augmentent. Or, on souhaite aussi avoir des produits les plus minces et les plus souples et conformables possibles pour s'adapter à l'anatomie de la zone du corps sur laquelle ils sont appliqués.

Il serait donc souhaitable de disposer d'articles qui permettent de gérer la propagation et le stockage des liquides, en particulier des liquides sécrétés par la plaie, la peau ou les muqueuses, en éloignant ces derniers de leur point de sécrétion pour éviter leur accumulation mais aussi leur migration latérale et leur retour vers la peau, la plaie ou les muqueuses. Dans le cas d'un pansement il serait de plus souhaitable, dans une version optimale, que ce dernier afin d'être le plus mince et conformable possible puisse s'affranchir des inconvénients liés à la présence d'une couche de stockage ce qui permettrait de diminuer, voire de supprimer l'obligation de le changer périodiquement.

L'invention vise précisément à réaliser des articles et en particulier des pansements qui répondent à ces objectifs.

L'invention cherche également à perfectionner des articles d'hygiène autres que des pansements, tels que des couches culottes ou articles d'hygiène féminine, en offrant une capacité d'absorption des fuites corporelles élevée, sans que cette capacité d'absorption nuise au confort de l'utilisateur.

La demande US2004/0127833 divulgue un procédé de fabrication d'un pansement, comportant une structure absorbante dans laquelle sont formées des cavités discrètes qui reçoivent un matériau absorbant. Ces cavités ne visent qu'à renforcer la capacité de stockage du pansement, et ne permettent pas de répondre de façon satisfaisante aux objectifs indiqués ci-dessus.

Le brevet US 4 676 785 décrit un article dans lequel le gonflement d'un matériau suite à l'introduction, côté proximal, d'un liquide depuis une ouverture, amène un clapet à fermer cette ouverture. Les cellules contenant le matériau hydrogonflable sont portées par un film de base imperméable qui constitue une structure distale par rapport à la zone d'émission du liquide. Il n'y a pas de transfert de liquide depuis la face de l'article située du côté d'émission du fluide vers la structure distale.

La demande EP 1 974 705 A1 décrit un article comportant une structure proximale en contact avec la peau et une structure distale formée d'un film imperméable. Il y a expansion suite à l'absorbtion d'un liquide, d'un matériau présent dans une cavité de l'article, mais cette expansion ne conduit pas au transfert de liquide à la structure distale.

Dans ces deux documents, le matériau apte à gonfler est présent à l'opposé de l'ouverture du liquide dans la cavité qui le contient.

La demande EP 0 875 222 décrit un article faisant intervenir une couche absorbante qui peut gonfler sous l'effet d'absorption d'eau, son expansion provoquant l'ouverture de fentes en contact avec une blessure.

Selon un premier de ses aspects, l'invention a pour objet un article selon les revendications 1 à 15, l'article ayant des propriétés d'évacuation et/ou de stockage d'un liquide, notamment un pansement, comportant :
- une structure proximale perméable au liquide, comportant un matériau capable de gonfler en présence du liquide,
- une structure distale apte à drainer un liquide et
- une structure intercalaire, de préférence ajourée, hydrophobe et non absorbante, s'étendant entre les structures distale et proximale, et étant apte d'une part à limiter localement, en l'absence de gonflement dudit matériau, l'échange de liquide entre les structures distale et proximale via le maintien d'un espacement entre ces structures, et d'autre part à autoriser une expansion locale de ladite structure proximale dans au moins une zone où le gonflement dudit matériau a lieu en réponse à une mise en présence avec un liquide, cette expansion conduisant au rapprochement local de la structure proximale et de la structure distale et au transfert dudit liquide de cette zone de la structure proximale ayant subi l'expansion à la structure distale.

En présence d'ajours dans la structure intercalaire, l'expansion peut avoir lieu au sein de ceux-ci, et un transfert par capillarité peut s'opérer par contact entre les structures proximale et distale à travers les ajours.

Selon l'invention, l'article est capable d'évacuer efficacement les sécrétions là où elles sont émises, en les drainant jusqu'à la structure distale. De plus, la région où l'article recouvre la peau, la plaie et/ou les muqueuses mais où il n'y a pas de sécrétion émise n'est pas affectée par le liquide présent dans la structure distale, car celui-ci ne peut redescendre vers la structure proximale en raison de la présence de la structure intercalaire.

A la différence des enseignements de US 4 676 785 et EP 1 974 705 A1, il peut y avoir un drainage du liquide jusqu'à une structure distale, où ce liquide peut continuer à être drainé. Ce drainage peut avoir lieu sans s'accompagner de la fermeture d'un clapet. Le matériau peut être situé initialement, dans l'article, plus proche de la surface d'admission du liquide dans l'article que du côté opposé.

Par « apte à drainer » on entend qualifier une capacité à stocker, étaler ou transférer par diffusion latérale, un liquide.

Par « diffusion latérale » il faut comprendre une diffusion dans une direction sensiblement perpendiculaire à la direction selon laquelle l'épaisseur de l'article est mesurée, soit sensiblement parallèlement à la surface sur laquelle est placé l'article lors de son utilisation.

Par « matériau capable de gonfler » il faut comprendre un matériau qui subit une augmentation de son volume en présence d'eau, par exemple d'au moins 10 %, mieux 200 %, voire 500 % ou 3000 %. Il peut s'agir d'un matériau composé d'un polymère superabsorbant ou comportant un tel polymère.

Par « matériau » on désigne un matériau unique ou un ensemble de matériaux différents qui sont associés ensemble, auquel cas le matériau est par exemple un matériau composite comportant un matériau superabsorbant mélangé à ou disposé entre un ou plusieurs autres matériaux, servant de support au matériau superabsorbant.

Le matériau capable de gonfler peut ainsi être un hydrocolloïde qui gonfle au contact des liquides, sous forme de poudre, de granulés ou de fibres, un matériau alvéolaire hydrogonflant comme par exemple une mousse de polyuréthane hydrophile ou un gel hydrophile comme par exemple un gel de polyuréthane hydrophile. Comme exemples d'hydrocolloïde on peut citer les dérivés de cellulose comme par exemple les sels de métal alcalin de la carboxyméthylcellulose, les alginates ou les polymères superabsorbants (SAP).

A titre de polymère superabsorbant (SAP), on peut utiliser un ou plusieurs polymères capables par exemple d'absorber de 10 à 50 fois leur poids d'eau (représentatif d'un liquide physiologique), et de ne pas relarguer l'eau en cas de pression modérée sur ceux-ci. La capacité d'absorption d'eau est due à l'interaction puissante entre les molécules d'eau et des groupements hydrophiles du polymère, notamment capables d'établir une liaison hydrogène. Les polymères superabsorbants peuvent être choisis notamment parmi les copolymères greffés à base d'amidon, les dérivés réticulés de carboxyméthylcellulose et les polyacrylates hydrophiles modifiés. Plus particulièrement, il peut s'agir de copolymères d'amidon et d'acrylonitrile hydrolysés, de copolymères d'amidon et d'acide acrylique neutralisés, de copolymères saponifiés, d'esters d'acide acrylique et vinylacétate, de copolymères d'acrylonitrile hydrolysés, de copolymères d'acrylamide, d'alcools polyvinyliques réticulés modifiés, de sels de polyacrylates réticulés, d'acide polyacrylique neutralisé et réticulé, de cellulose carboxylée ou un de leurs mélanges.

Généralement, ce matériau est mis en oeuvre à l'état pulvérulent et ce n'est qu'au contact d'un fluide, en l'occurrence, un liquide physiologique, qu'il va former un gel doté de propriétés de rétention.

A titre de matériaux superabsorbants préférés, on peut plus particulièrement citer les polyacrylates de sodium comme par exemple ceux commercialisés sous le nom de Favor®-Pac 230 ou Luquasorb® 1161.

A titre de matériau alvéolaire hydrogonflant, on peut également utiliser une mousse de polyuréthane hydrophile (PU) à l'image par exemple de celle commercialisée sous la dénomination MCF.03 par la société Advanced Medical Solution (AMS).

Comme matériau composite on peut citer des matériaux constitués des hydrocolloïdes cités précédemment incorporés dans une matrice à base d'une formulation à base de polymères, comme par exemple les compositions adhérentes hydrocolloïdes utilisées dans le domaine des pansements ou de la stomie, ou un matériau textile comme par exemple les non tissés absorbants incorporant des particules de SAP, couramment utilisés dans le domaine de l'hygiène.

On préfère l'utilisation de non tissés obtenus par la méthode de fabrication par voie sèche connue sous le nom de voie aérodynamique ou « airlaid » qui contiennent des particules de SAP et en particulier entre 20 à 60 % en poids de SAP par rapport au poids total du non tissé. De tels non tissés sont par exemple commercialisé par la société EAM Corporation sous la référence Novathin®. Selon un mode préféré de mise en oeuvre de la présente invention, on utilise un non tissé à base de particules de polymères superabsorbants et de fibres de cellulose sans incorporation de matériaux thermoliants ou de latex et qui est recouvert sur chacune de ses faces par un voile cellulosique.

Selon une autre variante de la présente invention, on peut aussi employer comme matériau composite un matériau constitué de deux voiles cellulosiques entre lesquels sont incorporées des particules de polymères super-absorbants, seules ou en association avec des liants.

Selon les applications de la présente invention on préfère employer un matériau capable de gonfler qui possède de propriétés de gonflement réversibles, c'est à dire qui, lors du séchage, se rétracte et retrouve par exemple sensiblement son volume initial, comme par exemple une mousse polyuréthane hydrophile ou un matériau à base de particules de SAP.

D'une façon générale, lors de l'utilisation au sein d'un pansement, on choisit un matériau compatible avec une stérilisation et convenant à cette utilisation, notamment du point de vue de son innocuité.

Il est souhaitable que la structure proximale soit conçue de telle sorte que le liquide ne migre pas (ou peu) latéralement dans celle-ci depuis la ou les zones où les sécrétions sont émises, afin que le gonflement dudit matériau n'ait lieu essentiellement qu'en regard de la ou des zones où les sécrétions sont émises.

La structure proximale a une répartition inhomogène du matériau capable de gonfler, notamment une répartition matricielle selon son plan, par exemple avec un intervalle allant de 1 mm à 20 mm entre les régions où le matériau capable de gonfler est présent. La répartition du matériau capable de gonfler est discrète, c'est à dire que les régions où le matériau capable de gonfler est présent ne se touchent pas.

Le matériau capable de gonfler peut être maintenu de diverses façons sur l'article. A cet effet, il est préférable que la structure proximale comporte un substrat perméable, qui sert de couche support au matériau capable de gonfler. Cette couche de support perméable doit limiter, voire éviter, la migration latérale des exsudats. Elle sert aussi de couche d'interface, destinée à venir en contact de la peau, de la plaie ou des muqueuses.

D'une façon générale, la couche support comprend un matériau perméable, mais peu ou non absorbant.

A titre de matériaux perméables on peut citer des matériaux textiles non absorbants comme les tricots, les tissés, les non tissés. On utilisera de préférence des non tissés non absorbants. Le non tissé peut être tout type de non tissé couramment utilisé dans le domaine de l'hygiène et des pansements, notamment un non tissé filé lié (spun laid), cardé (carded) ou hydrolié (spun lace) couramment désigné sous les termes de voile ou de « coverstock ». Son grammage est de préférence compris entre 5 et 50 g/m², de préférence entre 20 et 40 g/m². Le matériau textile est non absorbant en ce sens qu'il ne contient pas de fibres absorbantes telles que la rayonne, la viscose, les dérivés de cellulose, et qu'il ne contient pas de particules absorbantes. Il peut comprendre par exemple des fibres de polyamide, de polyester et ou de polyoléfines.

Le voile peut être hydrophile ou hydrophobe mais on préférera un voile hydrophobe. Selon un mode de réalisation, le voile comprend des fibres de polyéthylène. On choisira par exemple un non tissé « spunlaid », de préférence de type spunbond hydrophobe commercialisé par la société Fiberweb sous la dénomination Berotex® PE-SX ou un non tissé cardé hydrophile comprenant des fibres de polyester et de polyéthylène commercialisé par la société Sandler sous la dénomination Sawabond ® 4383.

Le voile non absorbant est de préférence constitué de fibres hydrophobes, mais il peut aussi être constitué de fibres hydrophiles et avoir subi un traitement pour le rendre hydrophobe. A l'inverse il peut être constitué de fibres hydrophobes et subir un traitement pour le rendre hydrophile. Le voile peut être constitué de plusieurs couches dans la mesure où sa perméabilité est suffisante.

La couche support peut être constituée d'un matériau unique ou d'une juxtaposition de matériaux différents, auquel cas on parle de matériau composite.

Comme autres matériaux perméables on peut citer des matériaux perforés ou microperforés comme des films plastiques (par exemple à base de polyuréthane ou de polyéthylène) perforés, des films 3D tels les produits commercialisés par la société TREDEGAR Film products. Ces matériaux sont bien connus de l'homme de l'art et couramment utilisés dans le domaine de l'hygiène. On peut aussi utiliser comme matériaux perméables des pansements connus sous le nom de "pansements interface", comme par exemple les produits commercialisés par les société Laboratoires URGO et MOLNLYCKE HEALTH CARE respectivement sous les dénominations URGOTUL® et MEPITEL®.

On peut enfin utiliser des couches perforées de formulations hydrophobes ou hydrophiles mais non absorbantes ou peu absorbantes, à base de polymères. Ces formulations pourront être adhérentes ou non adhérentes. Dans le cas de pansements, on préférera utiliser des formulations microadhèrentes ou non adhérentes, qui permettent de ne pas altérer le processus de cicatrisation lors du retrait de la plaie.

De telles formulations sont bien connues de l'homme de l'art et sont par exemple réalisées à base de gel(s) de silicone, d'adhésif(s) siliconé(s) sensibles à la pression ou de compositions contenant un élastomère séquencé du type poly ( styrène - oléfine - styrène), un plastifiant tel une huile minérale et une faible quantité d'hydrocolloïde(s) pour créer un environnement humide pour favoriser le processus de cicatrisation sans rendre la composition absorbante pour éviter de boucher les trous. De telles formulations micro adhérentes sont par exemple utilisées dans les pansements commercialisées par la société Laboratoires URGO sous les dénominations URGOCLEAN® et URGOTUL ABSORB®.

Selon des variantes possibles, ces pansements interfaces et ces couches perforées de formulations hydrophobes ou hydrophiles pourront être associés avec les matériaux perméables préalablement cités, en particulier les non tissés non absorbants.

Dans un exemple de mise en oeuvre de l'invention, le matériau capable de gonfler est supporté par une couche de support perméable, de préférence hydrophobe comme détaillé ci-dessus, et est par exemple fixé sur la face de cette couche qui est tournée vers la structure intercalaire. Dans une variante, le matériau capable de gonfler est présent entre deux couches de la structure proximale, et y est par exemple prisonnier de cavités formées par l'assemblage de ces couches.

Le matériau capable de gonfler peut être intégré à l'article, au cours de sa fabrication, sous une forme non pulvérulente, par exemple sous forme d'inserts tels que des pastilles ou agrégats, dont la plus grande dimension est par exemple supérieure ou égale à 1 mm et inférieure ou égale à 15 mm. Ces inserts peuvent, le cas échéant, être en un matériau composite comportant un polymère superabsorbant sous forme pulvérulente. Dans un exemple de mise en oeuvre de l'invention, le matériau capable de gonfler est ainsi incorporé à l'article après découpage dans une feuille, par exemple par une opération de poinçonnage qui permet de former les pastilles précitées, celles-ci étant par exemple de contour circulaire ou polygonal, de préférence régulier.

Dans d'autres exemples de mise en oeuvre de l'invention, le matériau capable de gonfler est incorporé à l'article, durant sa fabrication, sous forme pulvérulente, de préférence avec une répartition non homogène, par exemple sous forme d'agglomérats localisés comme les ajours de la structure intercalaire.

La structure proximale peut comporter au moins une couche de support, comme décrite ci-dessus, pour assurer le maintien du matériau capable de gonfler sur l'article, la ou les couches de support étant réalisées de façon à ne pas contrarier l'expansion du matériau capable de gonfler en direction de la structure distale, tout en contrariant de préférence l'expansion dans la direction opposée. En variante, la structure proximale est constituée uniquement par le matériau capable de gonfler, la fonction de support du matériau capable de gonfler étant assurée par la structure intercalaire. Dans ce cas notamment, le matériau capable de gonfler peut être incorporé sous une forme discontinue dans l'article, par exemple sous la forme d'inserts tels que des pastilles logées dans des ajours de la structure intercalaire. Dans ce cas notamment, la structure intercalaire peut venir au contact de la peau, la plaie ou des muqueuses. De préférence, l'article, notamment dans le cas d'un pansement, comporte une couche d'interface avec la plaie qui est continue et s'étend sur toute la face inférieure de l'article susceptible de contacter la plaie. Cette couche d'interface peut servir ou non à maintenir le matériau capable de gonfler sur l'article. La couche d'interface peut être utile pour limiter, voire éviter, l'expansion de la structure proximale en direction de la peau et/ou des muqueuses. Les inserts peuvent être portés par la couche d'interface alors que celle-ci est disposée contre la structure intercalaire.

La structure proximale peut être entièrement située hors de la structure intercalaire en l'absence de gonflement du matériau apte à gonfler. En variante, la structure proximale est engagée dans la structure intercalaire en l'absence de gonflement du matériau apte à gonfler, s'étendant sur une partie seulement de l'épaisseur de celle-ci.

La structure proximale comporte par exemple le matériau apte à gonfler sous forme d'inserts, celui-ci étant de préférence un non-tissé dans lequel sont emprisonnées des particules d'un superabsorbant, la structure intercalaire comportant des ajours dans lesquels sont engagés au moins partiellement les inserts, la structure intercalaire ayant une épaisseur suffisante pour qu'avant gonflement les inserts ne contactent pas la structure distale, la structure intercalaire ayant notamment une épaisseur supérieure à celle des inserts, et suffisamment faible pour que le gonflement des inserts les amène à contacter la structure distale.

La structure intercalaire est hydrophobe et non absorbante, de façon à isoler sur le plan fluidique les structures proximale et distale là où aucune expansion de la structure proximale n'a lieu. Elle doit aussi éviter la migration latérale du liquide et dans le cas où elle est ajourée, empêcher le liquide de passer latéralement d'un ajour à l'autre. De préférence, elle est souple pour pouvoir se conformer aux contours anatomiques. La structure intercalaire est par exemple un matériau alvéolaire à cellules fermées sous la forme d'une couche d'une mousse d'un matériau thermoplastique, notamment une polyoléfine, par exemple du polyéthylène, ou un assemblage de plusieurs couches de ce type. Elle peut aussi se présenter sous la forme d' un film , d'un matériau textile ou d'une couche, hydrophobes et non absorbants, à base d'adhésifs, de polymères ou d'élastomères tels le polyuréthane, le polydimèthylsiloxane et ses déclinaisons ( désignés généralement sous le terme générique de « silicone »), les polyoléfines (comme par exemple le polyéthyléne) le polyphénylèneéther ou des formulations à base de polymères séquencés par exemple du type( styrène - oléfine - styrène ) ou (styrène - oléfine) associés à un plastifiant.

L'épaisseur de la structure intercalaire est par exemple comprise entre 0,5 mm et 4 mm pour un article constituant un pansement. Lorsque le matériau capable de gonfler est présent sous la forme d'inserts au sein de la couche intercalaire, l'épaisseur de celle-ci est supérieure à celle des inserts.

La structure intercalaire est de préférence ajourée, comme mentionné précédemment, et l'expansion de la structure proximale a alors lieu au moins partiellement dans un ajour de la structure intercalaire. Ainsi, la répartition des ajours de la structure intercalaire est avantageusement sensiblement la même que celle du matériau capable de gonfler, de façon à ce que les régions où ce matériau est présent puissent se superposer aux ajours, de préférence d'une manière centrée au sein de chaque ajour. Les ajours peuvent avoir toute forme. Les ajours peuvent être formés par découpe dans une feuille, en ligne au cours de la fabrication de l'article, ou être découpés lors d'une opération préalable. Les ajours peuvent avoir tous le même contour ou non, avec par exemple une répartition des ajours qui est régulière ou non. Les ajours peuvent être de contour circulaire ou polygonal, notamment polygonal régulier. Les ajours peuvent être de section constante sur toute l'épaisseur de la structure intercalaire, ou en variante avoir une section qui varie, notamment décroît en direction de la structure proximale, de façon à favoriser l'expansion de celle-ci en direction de la structure distale plutôt que dans la direction inverse. Pour obtenir une section qui décroît, on peut utiliser par exemple plusieurs feuilles dans lesquelles les ajours sont découpés avec des tailles décroissantes, puis procéder à l'assemblage desdites feuilles. On peut encore découper les ajours directement avec la forme souhaitée, par exemple en utilisant un laser.

La structure distale est apte à drainer les liquides c'est à dire qu'elle permet de les stocker, de les transférer et ou les étaler.

Dans un exemple de mise en oeuvre de l'invention, la structure distale comporte une couche formant réservoir, ayant une capacité d'absorption d'eau supérieure ou égale à 500 g/m² voire 800g/m². La couche formant réservoir peut se superposer à la structure intercalaire. La couche formant réservoir peut être constituée de tout matériau apte à stocker les liquides comme par exemple les couches absorbantes couramment utilisées dans le domaine de l'hygiène et des pansements. On peut citer à titre d'exemple, les mousses absorbantes et de préférence les mousses polyuréthane hydrophiles et tous les matériaux à base de SAP précédemment cités, les textiles absorbants comme par exemple les non tissés à base de viscose, de rayonne ou de cellulose, comme par exemple une ouate ou des hydrogels.

Dans un autre exemple de mise en oeuvre de l'invention, la structure distale est dépourvue d'une couche formant réservoir ayant une capacité d'absorption d'eau supérieure ou égale à 500g/m². Dans ce cas la couche distale est constituée d'une couche qui permet une diffusion latérale du liquide pour l'étaler ou favoriser son transfert. Le liquide est alors, par exemple, évacué vers une couche formant réservoir située en périphérie de la structure intercalaire ou vers une partie terminale libre où le liquide peut s'évaporer.

Lorsque la couche formant réservoir est située au moins partiellement sur le côté de la structure intercalaire, on peut obtenir un article moins épais, à capacité d'absorption équivalente. Dans un exemple de mise en oeuvre, la couche formant réservoir s'étend tout autour de la structure intercalaire. L'article comporte avantageusement, lorsque la couche formant réservoir est située sur le côté de la couche intercalaire, une couche formant barrière située entre la peau et/ou les muqueuses et la couche formant réservoir, qui est imperméable à l'eau. Lorsque la couche formant réservoir est située sur le côté de la structure intercalaire, le liquide est acheminé dans cette couche formant réservoir par une couche de transfert qui constitue tout ou partie de la structure distale. Une couche de protection extérieure peut recouvrir cette couche de transfert et la couche formant réservoir. De préférence on utilisera la combinaison d'une couche de diffusion latérale et d'une couche réservoir pour favoriser l'absorption du liquide par la couche réservoir grâce à une grande surface de contact entre ces deux couches.

Des matériaux permettant l'étalement et/ou le transfert des liquides par diffusion latérale sont couramment employés dans le domaine de l'hygiène et des pansements.

On peut ainsi citer des matériaux textiles tels les tricots, les tissés et tout particulièrement les non tissés. Ces matériaux textiles pourront être hydrophobes ou hydrophiles à base de fibres absorbantes ou non. Parmi les non tissés on préférera les non tissés hydrophiles et en particulier ceux à base de fibres absorbantes, telle que la viscose ou la cellulose, associées à des fibres non absorbantes comme par exemple des fibres de polyester ou de polyoléfines. A titre d'exemple de tels non tissés on peut citer les produits commercialisés respectivement par les sociétés Suominen Corp et Orsa sous les dénominations Fibrella® 2000 et Jettex® 1205 c.

Comme autres matériaux, on peut aussi citer les papiers ou des films microstructurés dont les canaux permettent l'étalement et la migration des liquides.

La structure intercalaire peut assurer la fixation de la structure proximale sur la structure distale, notamment comporter ou se présenter sous forme d'une couche d'adhésif imperméable à l'eau, s'étendant de façon discontinue entre la structure distale et la structure proximale, de façon à ménager au moins un ajour dépourvu d'adhésif où le gonflement du matériau de la structure proximale conduit à un transfert par capillarité du liquide vers la structure distale par contact entre la structure proximale et la structure distale sous l'effet dudit gonflement.

La structure distale peut comporter au moins une cavité débouchant en direction de la structure proximale, cette cavité se superposant au moins partiellement à une zone de la structure proximale, où le gonflement est susceptible d'avoir lieu, et de préférence se superposant au moins partiellement à un ajour de la structure intercalaire. La présence d'une telle cavité peut permettre d'éloigner localement la structure proximale de la structure distale en l'absence de gonflement et peut permettre de réduire l'épaisseur de la structure intercalaire ou d'accroître celle de la structure proximale là où le matériau capable de gonfler est présent.

La structure intercalaire peut comporter deux couches ajourées, dont une couche inférieure du côté de la structure proximale et une couche supérieure du côté de la structure distale, les ajours de la couche supérieure ayant une section moindre que les ajours de la couche inférieure, et se superposant à ces derniers. Le matériau capable de gonfler peut être contenu au moins partiellement, lorsque sec, dans les ajours de la couche inférieure. Le matériau capable de gonfler peut ou non remplir en totalité les ajours de la couche inférieure. Une telle variante peut faciliter le maintien du matériau capable de gonfler. De plus, une forme étagée de la cavité contenant le matériau capable de gonfler peut accroître la sensibilité à l'eau de l'interrupteur fluidique formé avec ce matériau, car à volume d'expansion égal du matériau par rapport à une cavité de section constante et de même hauteur et avec la même quantité de matériau initiale, la présence d'une partie supérieure plus étroite permet d'accroître la distance selon laquelle le matériau se déplace en direction de la structure distale, et donc de contacter plus vite celle-ci.

Les ajours de la couche inférieure ont de préférence une plus grande dimension, notamment un diamètre, compris entre 5 et 25 mm, et les ajours de la couche supérieure ont de préférence une plus grande dimension, notamment un diamètre, compris entre 1 et 10 mm

De préférence, le matériau capable de gonfler est retenu dans les ajours de la couche inférieure sans utilisation d'adhésif du côté de la structure proximale.

Le matériau capable de gonfler peut avantageusement être pâteux, au moins lors de la confection de l'article, notamment lors de son introduction dans des ajours de la structure intercalaire. Cela peut faciliter sa mise en place dans les ajours par étalement et raclage.

Le matériau capable de gonfler peut comporter un polymère hydroexpansible, notamment à base de particules de SAP et un liant hydrosoluble, notamment à base de polyvinylpyrrolidone et/ou d'hydroxypropylcellulose.

Le matériau capable de gonfler peut notamment avoir la formulation suivante, exprimée en masse par rapport à la masse totale du mélange :
- de 10 à 90 % de polymère(s) hydroexpansible(s), notamment à base de particules de SAP,
- 1 à 20% de liant(s) hydrosoluble(s), par exemple à base de polyvinylpyrrolidone et/ou d'hydroxypropylcellulose,
- de 0 à 20% de glycérine, et
- de 30 à 80 % de liquide d'homogénéisation, notamment à base d'alcool, de préférence de l'éthanol.

La structure distale peut comporter une pluralité de cavités disposées selon une répartition matricielle, les cavités étant de préférence réparties comme les zones de la structure proximale susceptibles de gonfler. La profondeur de la ou des cavités est de préférence inférieure à l'épaisseur de la structure distale, et comprise par exemple entre 10 et 90 % de l'épaisseur de la structure distale.

L'article constitue de préférence un pansement, conditionné à l'état stérile, mais en variante peut aussi constituer une couche culotte ou un article d'hygiène féminine. Lorsque l'article est un pansement, hormis les trois structures précédemment définies (structure proximale, structure intermédiaire, structure distale), le pansement comporte de préférence des couches supplémentaires pour garantir son asepsie avant et durant usage. Ainsi, côté plaie, selon la nature de la couche d'interface, en particulier si elle est à base d'un pansement interface ou une formulation à base de polymères, il pourra comprendre un protecteur provisoire pouvant être retiré avant usage. A l'opposé il est recouvert d'une couche imperméable aux bactéries et à l'eau mais perméables à la vapeur d'eau de manière à favoriser l'évaporation des liquides, dite couche de protection extérieure. De telles couches sont couramment utilisées dans la réalisation de pansements et sont par exemple constituées de films de polyuréthane tels les films commercialisés par la société Exopack Advanced Coating sous la désignation INSPIRE. Un tel film peut être assemblé au pansement à l'aide par exemple d'un adhésif discontinu de manière à ne pas affecter la perméabilité du film aux gaz et en particulier à la vapeur d'eau. Il peut également être assemblé à la structure proximale en périphérie du pansement.

Dans la version où la bande de transfert a une longueur supérieure à celle de la structure intercalaire, cette dernière est avantageusement enveloppée d'une telle couche de protection extérieure, pour éviter que les exsudats présents dans la couche souillent les alentours de la plaie et également de prévenir les risques de contamination extérieure du pansement par les bactéries.

Ainsi, selon l'un de ses aspects, l'invention concerne un dispositif pour transférer un liquide, comportant :
- une structure proximale, perméable au liquide, destinée à venir au contact du liquide,
- une structure distale, perméable au liquide, et
- une structure intercalaire hydrophobe et non absorbante, disposée entre la structure proximale et la structure distale,
ladite structure proximale comprenant un matériau susceptible de gonfler sous l'effet des liquide, de telle sorte que le gonflement dudit matériau réduise la distance entre la structure proximale et la structure distale, le liquide étant alors susceptible de passer de la structure proximale vers la structure distale, à travers la structure intercalaire.

L'invention pourra être mieux comprise à la lecture de la description détaillée qui va suivre, d'exemples de mise en oeuvre non limitatifs de celle-ci, et à l'examen des dessin annexés, sur lesquels :
- la figure 1 représente de façon schématique et partielle un exemple d'article réalisé conformément à l'invention,
- la figure 2 est une coupe selon II-II de la figure 1,
- la figure 3 représente un détail de la figure 1,
- la figure 4 illustre l'utilisation de l'article des figures 1 à 3 en place sur une plaie,
- les figures 5 à 8 sont des vues analogues à la figure 1 de variantes de réalisation,
- la figure 9 est une coupe selon IX-IX de la figure 8,
- la figure 10 est une vue analogue à la figure 1 d'une variante de réalisation,
- la figure 11 illustre l'utilisation de l'article de la figure 10,
- la figure 12 est une vue analogue à la figure 1 d'une variante de réalisation d'article selon l'invention,
- la figure 13 illustre l'utilisation de l'article de la figure 12,
- la figure 14 est une vue analogue à la figure 3 d'une variante de réalisation,
- la figure 15 est une vue analogue à la figure 1 d'une variante de réalisation, et
- les figures 16 et 17 représentent respectivement, en vue de dessus, les couches ajourées de la structure de la figure 15.

Sur les figures, les proportions réelles des différents éléments constitutifs n'ont pas toujours été respectées, dans un souci de clarté du dessin. De même, certains éléments ont pu être représentés avec un écartement alors qu'ils sont dans la réalité en contact.

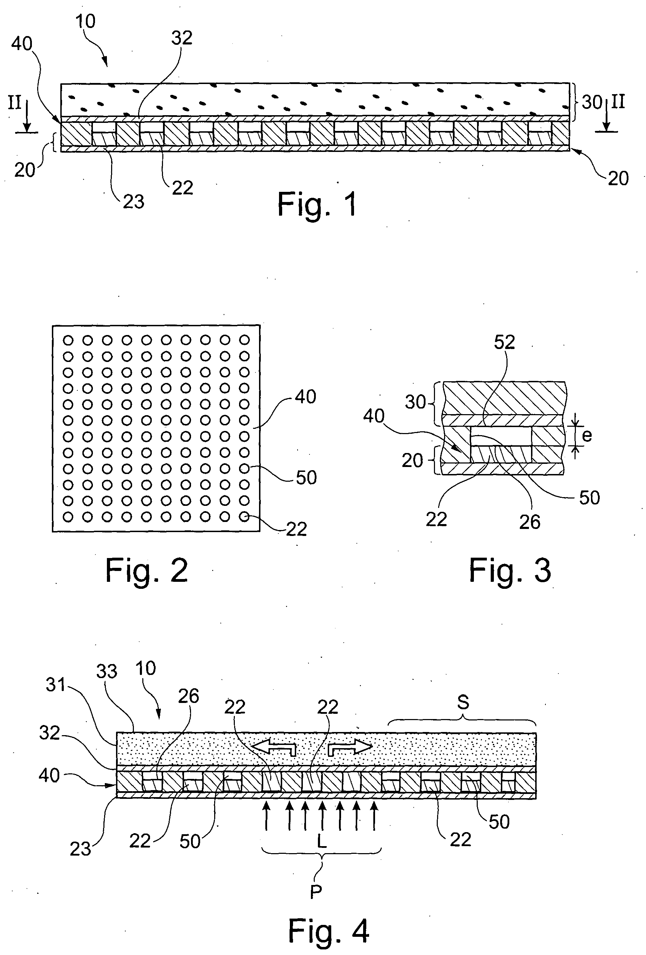

L'article 10 selon l'invention représenté sur les figures 1 à 4 est un pansement destiné à être appliqué sur une plaie, mais toute la description qui suit vaut pour des articles autres qu'un pansement, notamment pour un article d'hygiène féminine ou une couche culotte.

Dans un souci de simplification le pansement n'a pas été représenté avec une couche de protection extérieure sur toutes les figures.

L'article 10 comporte une structure proximale 20 destinée à contacter la peau et la plaie, en étant de préférence centrée sur la plaie, une structure distale 30 et une structure intercalaire 40.

Chacune des structures proximale, distale et intercalaire peut comporter une ou plusieurs couches constitutives, assemblées entre elles de façon permanente ou non, à l'aide d'adhésif et/ou par fusion locale de matière. Lorsqu'une couche d'adhésif est utilisée, celle-ci n'a pas toujours été représentée sur le dessin, dans un souci de clarté.

La structure distale 30 est réalisée dans l'exemple des figures 1 à 4 de façon à absorber le liquide et à le stocker, avec une capacité d'absorption relativement élevée et notamment supérieure à 500 g/m², voire 800g/m². En variante, la structure distale 30 est réalisée de façon à permettre la migration latérale du liquide. Cette migration peut s'effectuer vers une région de l'article où le liquide peut être évacué par évaporation, et/ou vers un réservoir qui est situé sur le côté de l'article, comme cela sera détaillé par la suite.

La structure intercalaire 40 permet de limiter l'échange de liquide entre les structures distale 30 et proximale 20. L'article est agencé pour favoriser l'échange de liquide dans la ou les zone(s) de l'article qui se superpose(nt) à la ou aux zone(s) P de sécrétion de liquide, et pour réduire, mieux éliminer, l'échange de liquide depuis la structure distale vers la structure proximale dans la ou les zone(s) S qui sont décalées latéralement par rapport à la ou aux zones P de sécrétion du liquide.

La structure intercalaire 40 peut ainsi être réalisée de façon à maintenir un espacement entre les structures proximale 20 et distale 30 ailleurs qu'à l'aplomb de la ou des zones P de sécrétion du liquide.

Dans l'exemple des figures 1 à 4, la structure intercalaire 40 comporte des ajours 50, notamment disposés comme les trous d'une grille, avec un écartement constant entre les ajours dans deux directions perpendiculaires entre elles.

Hors les ajours 50, la structure intercalaire 40 vient au contact de la structure distale 30 selon des régions d'assemblage 52 où la structure intercalaire 40 est par exemple fixée à la structure distale 30 par collage ou soudure.

La structure proximale 20 comporte un matériau capable de gonfler en présence d'eau. Ce matériau est par exemple, comme illustré, présent sous la forme d'inserts 22 engagés dans les ajours 50. Le gonflement des inserts 22 qui se produit dans les ajours 50 en présence du liquide sécrété permet à la structure proximale 20, dans l'exemple considéré, de subir une expansion au point de contacter la structure distale 30, comme illustré à la figure 4. Ce contact crée un pont fluidique qui permet une diffusion du liquide depuis la structure proximale dans la structure distale 30.

Pour fabriquer l'article 10, on peut réaliser la structure intercalaire 40 par exemple sous la forme d'une grille en poinçonnant une feuille d'un matériau hydrophobe et non absorbant de façon à réaliser les ajours 50.

Les inserts 22 peuvent être réalisés par découpage d'une feuille d'un matériau ayant une capacité de gonflement en présence d'eau, en donnant à chaque insert une forme complémentaire à celle d'un ajour ou à tout le moins permettant à l'insert 22 de se loger au moins partiellement dans l'ajour 50. L'épaisseur des inserts 22 étant inférieure à celle des ajours 50, on obtient un espacement *e* non nul entre les faces libres 20 des inserts 22 et la structure distale 30, une fois l'article 10 assemblé.

Les inserts 22 sont de préférence, comme illustré, supportés une couche d'interface 23 avec la peau, la plaie et/ou les muqueuses, s'étendant sous la structure intercalaire 40.

Lors de la fabrication, les inserts 22 sont par exemple mis en place sur la couche d'interface 23 avant assemblage de celle-ci avec la structure intercalaire 40.

La structure distale 30 comporte par exemple, comme illustré, une couche formant réservoir 31 de capacité d'absorption supérieure ou égale à 500 g/m², située entre une couche de transfert 32 et une couche extérieure de protection 33 qui est, par exemple, imperméable à l'eau mais perméable à la vapeur d'eau.

La couche de transfert 32 peut être collée ou assemblée par d'autres moyens à la structure intercalaire 40.

Lors de l'utilisation de l'article 10, la sécrétion du liquide L dans au moins une zone P sur laquelle est placé l'article 10 entraine un gonflement du ou des inserts 22 qui se superposent à cette zone P.

Le gonflement de ces inserts 22 les amène à s'expanser sur toute l'épaisseur de la structure intercalaire 40 et à contacter la structure distale 30, en l'espèce la couche de transfert 32, ce qui conduit localement à la création d'un pont fluidique entre la structure proximale 20 et la structure distale 30, comme illustré. Ce pont fluidique permet un transfert de liquide depuis la structure proximale 20 vers la structure distale 30, et permet dans l'exemple considéré à la couche formant réservoir 31 d'absorber le liquide sécrété. Celui-ci peut ensuite diffuser latéralement dans la structure distale 30 vers au moins une zone S, décalée latéralement de la zone P.

Le liquide qui s'accumule dans la zone S ne peut pas retourner vers la peau, la plaie et/ou les muqueuses en redescendant verticalement à travers l'article 10, du fait de la présence de l'espace rempli d'air dans les ajours 50 entre les inserts 22 qui n'ont pas subi de gonflement et la couche de transfert 32. L'article 10 permet ainsi de maintenir la surface de la peau la plaie et/ou des muqueuses située dans la zone S au sec.

On peut utiliser pour réaliser la couche d'interface 23 un tissu à mailles ouvertes enduit d'un gel formé d'une matrice élastomérique hydrophobe fortement plastifiée et contenant une faible quantité de particules d'un hydrocolloïde, tel que décrit dans l'exemple 1 de la demande internationale WO 0016725.

Les inserts 22 sont par exemple réalisés à partir d'un matériau hydrogonflant en feuille, ayant par exemple une épaisseur de l'ordre de 1 mm, voire du dixième de mm, et sont disposés sur la face interne de la couche d'interface 23, opposée à celle destinée à venir en contact avec la plaie.

On peut utiliser pour réaliser les inserts 22 un matériau en feuille de marque NOVATHIN® commercialisé par la société EAM sous la référence J4000950DTNB, qui est un substrat de fibres de cellulose et de polymère superabsorbant ayant un grammage de 400 g/m² et une épaisseur de 1,14 mm, ou le matériau en feuille commercialisé par la société BUCKEYE STEINFURT GmbH sous la référence Vizorb® 3924 (180MBS3A), de grammage d'environ 180 g/m².

Pour constituer la structure intercalaire 40, on peut assembler plusieurs couches fines de mousse à cellules fermées, par exemple 3 à 4 couches, afin d'atteindre une épaisseur supérieure ou égale à 3 mm. L'assemblage s'effectue par lamination par exemple à l'aide d'un adhésif double face. La structure intercalaire 40 est par exemple constituée par trois couches d'une mousse hydrophobe à cellules fermées en polyéthylène (PE) de marque Alveolit® TEE.1000.8 de 0.8 mm d'épaisseur. Les ajours 50 sont par exemple constitués de trous circulaires de 5 mm de diamètre, dont les centres sont espacés régulièrement de 1 cm dans les deux directions.

La couche de transfert 32 est constituée par exemple par un non-tissé JETTEX® 1205 C de la société ORSA et la couche formant réservoir 31 est par exemple constituée par une mousse hydrophile, par exemple une mousse de PU hydrophile de référence MCF 03 et de 4,5 mm d'épaisseur de la société AMS.

Pour assembler la couche d'interface 23 avec la structure intercalaire 40, on peut par exemple chauffer l'ensemble sous pression.

Pour effectuer le collage de la couche de transfert 32 sur la structure intercalaire 40 on peut par exemple utiliser un adhésif double face.

Le collage de la couche formant réservoir 31 peut s'effectuer par exemple à l'aide d'un voile thermoliant 65-100°C.

Dans une variante de réalisation, on remplace la couche d'interface 23 par un non-tissé hydrophobe du type coverstock (Berotex® PE-SX), précédemment décrit.

### Essais comparatifs

Les essais sont effectués à 23 °C et 33/35 % d'hygrométrie.

On compare un pansement connu de référence URGOTUL ABSORB® et un pansement selon l'invention ayant la structure illustrée aux figures 1 à 4 et réalisé avec les matériaux suivants :
- couche d'interface 23 : selon exemple 1 de WO 00/16725 tricot thermofixée en fils de polyester enduite d'un gel formé d'une matrice élastomérique de S-EB-S à haut poids moléculaire, fortement plastifiée et contenant en dispersion, environ 15 % en poids par rapport à la masse de gel, de particules hydrophiles d'un hydrocolloïde constitué de carboxyméthylcellulose sodique,
- inserts 22 : pastilles de NOVATHIN® J4000950DTNB dont l'épaisseur est environ 1 mm,
- structure intercalaire 40 : mousse hydrophobe de PE à cellules fermées Alveolit® de 3 mm d'épaisseur, perforée comme sur la figure 2, avec des trous circulaires de 5 mm de diamètre dont les centres sont espacés de 10 mm,
- couche de transfert 32 : non-tissé à base de cellulose (55 %) et de Polyester (45 %), JETTEX® 1205C de la société ORSA,
- couche formant réservoir 31 : mousse de PU hydrophile de 4,5 mm d'épaisseur de référence MCF 03 du fabricant AMS.

Au niveau des inserts 22, un point d'encre peut être déposé, de façon à, après utilisation, déterminer quels sont ceux qui ont subi un gonflement et pour lesquels l'encre a été diluée.

Les pansements sont disposés chacun sur un pavé de verre fritté, par lequel est injectée une solution de chlorure de sodium à 0,83 % et de chlorure de calcium à 0,04 % (en poids).

Un volume de 10 ml est injecté dans le pansement par l'intermédiaire d'un pousse seringue relié à un pavé de verre fritté mis en contact avec la face inférieure du pansement, ce pavé de fer fritté simulant la plaie. Le pansement est positionné horizontalement avec un poids disposé à la surface du pansement exerçant une pression de 25 mbar. Le débit d'injection du liquide, à travers le pavé de verre fritté est fixé à 10 µl/mn.

A l'issue de l'expérimentation, seuls les inserts en contact de la plaie ont subi un gonflement : pour ces derniers, l'encre n'apparaît plus. Les inserts n'étant pas en contact de la plaie simulée conservent leur aspect initial. Dans le cas du pansement de référence (URGOTUL ABSORB), la surface humide en contact avec la peau est estimée à 90 cm², tandis que pour un pansement selon l'invention, la surface humide est estimée à seulement 7 cm², soit la surface du pavé de verre fritté, ce qui démontre l'effet vis-à-vis de la préservation de la peau périlésionnelle. Ainsi, grâce à l'invention, la surface humide en contact avec la peau reste centrée sur la plaie et l'on diminue, en outre, le risque de fuite, notamment lorsque le pansement n'est pas horizontal. Les résultats démontrent l'efficacité de l'invention, tant pour un pansement orienté horizontalement que pour un pansement orienté verticalement.

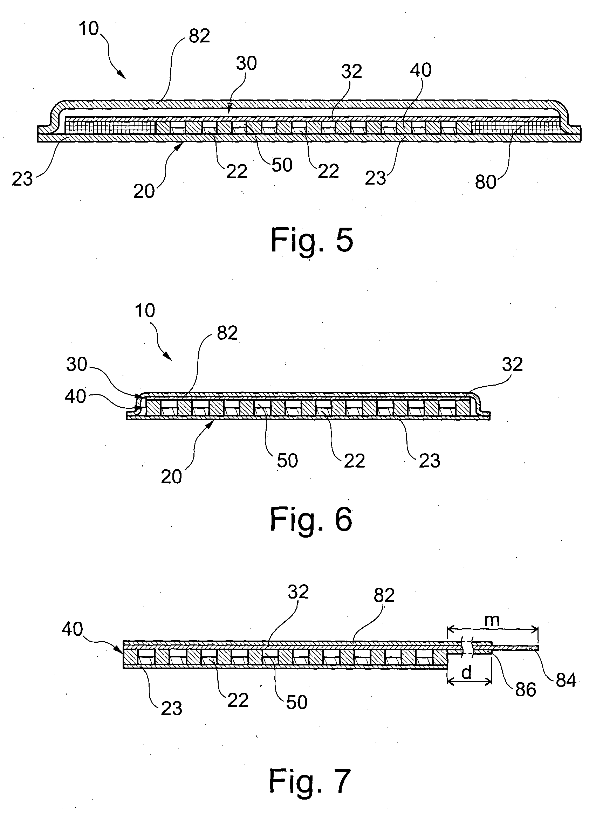

La variante de réalisation de l'article 10, représentée à la figure 5, diffère de celle décrite en référence aux figures 1 à 4 par le fait que la couche formant réservoir 31 superposée à la structure intercalaire 40 est remplacée par une couche formant réservoir 80 déportée latéralement et s'étendant par exemple sur tout le périmètre de la structure intercalaire 40.

La couche d'interface 23 peut être rendue imperméable à l'eau sous la couche formant réservoir 80, ou une couche formant barrière, non représentée, est introduite entre les deux.

La structure distale 30 est limitée dans l'exemple de la figure 5 à la couche de transfert 32, laquelle permet au liquide qui l'atteint de diffuser latéralement jusqu'à la couche formant réservoir 80.

Une couche extérieure de protection 82 peut recouvrir la couche formant réservoir 80 et être assemblée à sa périphérie avec la couche d'interface 23.

L'exemple de la figure 5 comporte le même agencement permettant un déclenchement sélectif de la communication fluidique que dans l'exemple des figures 1 à 4, c'est-à-dire que des inserts 22 sont disposés dans des ajours 50 formés au travers d'une structure intercalaire 40.

L'épaisseur de la couche formant réservoir 80 est par exemple, comme illustré, sensiblement égale à +/- 20 % à celle de la structure intercalaire 40, de façon à disposer d'un article 10 présentant une épaisseur sensiblement uniforme.

Au cours de l'utilisation, le liquide qui est sécrété par la plaie fait gonfler les inserts 22 qui se trouvent au-dessus de la plaie, permettant un contact entre ces inserts 22 et la couche de transfert 32, puis l'étalement du liquide au sein de celle-ci en direction de la couche formant réservoir 80, où le liquide peut s'accumuler.

Les inserts 22 qui ne se situent pas au-dessus de la source d'émission des sécrétions de liquide ne gonflent pas ou pas suffisamment pour contacter la couche de transfert 32, de telle sorte que le liquide qui diffuse dans cette couche de transfert 32 ne redescend pas vers la couche d'interface 23 en gagnant la couche formant réservoir 80.

La variante de réalisation de la figure 6 diffère de celles qui viennent d'être décrites en référence aux figures 1 à 5 par l'absence de couche formant réservoir 31 ou 80, l'évacuation du liquide s'effectuant essentiellement par évaporation. Dans cet exemple, la couche de protection 82 est perméable à la vapeur d'eau et assemblée à sa périphérie avec la couche d'interface 23.

Dans la variante de réalisation illustrée à la figure 7, la couche de transfert 32 se prolonge latéralement, en porte à faux, au-delà de la structure intercalaire 40, par exemple sur une distance *m* égale à 20 cm, et forme une partie terminale libre 84 au contact de l'air ambiant.

La couche de protection extérieure 82 s'étend sur une distance *d* inférieure à *m*, par exemple d'environ 10 cm, dans une maquette réalisée, et ne recouvre supérieurement la couche de transfert 32 que sur une portion de sa longueur, de façon à laisser libre la partie terminale 84 et à faciliter l'évaporation de l'eau à son niveau. Une couche de protection intérieure 86 peut être présente sur la couche de transfert 32 du côté de sa face inférieure, de façon à éviter tout contact de la couche de transfert 32 avec la peau et/ou les muqueuses à proximité de la plaie.

Lors de l'utilisation de l'article de la figure 7, le liquide contacte localement la couche de transfert 32 grâce aux ponts fluidiques qui se forment après gonflement des inserts 22 directement confrontés à la sécrétion du liquide, et migre vers la partie terminale 84, où l'eau peut s'évaporer.

Un volume de 30 ml est injecté dans le pansement par l'intermédiaire d'un pousse seringue relié à un fritté mis en contact avec la face inférieur du pansement.

Le débit d'injection du liquide est fixé à 10 µl/mn. Après 50 H de fonctionnement 28 ml du liquide injecté a été évaporé, soit un taux d'évaporation supérieur à 90 %.

Selon une variante de la présente invention, on incorpore au-dessus de la bande de transfert 32 ou du réservoir 80 une chambre permettant le passage d'air de préférence d'air sec, pour augmenter l'évaporation du liquide présent dans la structure distale.

Le type de pansement représenté sur la figure 6, ne comportant pas de couche réservoir au sens de la présente invention, est particulièrement adapté à des plaies à exsudation modérée ou faible, par exemple en cours d'épidermisation.

Pour des plaies plus exsudatives, on préfère l'utilisation d'un pansement comportant une couche réservoir, ou un pansement sans couche absorbante, tel que représenté sur la figure 7, le débordement de la bande de transfert permettant d'optimiser l'évaporation.

On voit qu'on peut ainsi disposer d'une gamme de pansements, avec ou sans couche réservoir, adaptée à plusieurs types de plaies, selon qu'elles soient exsudatives ou peu exsudatives.

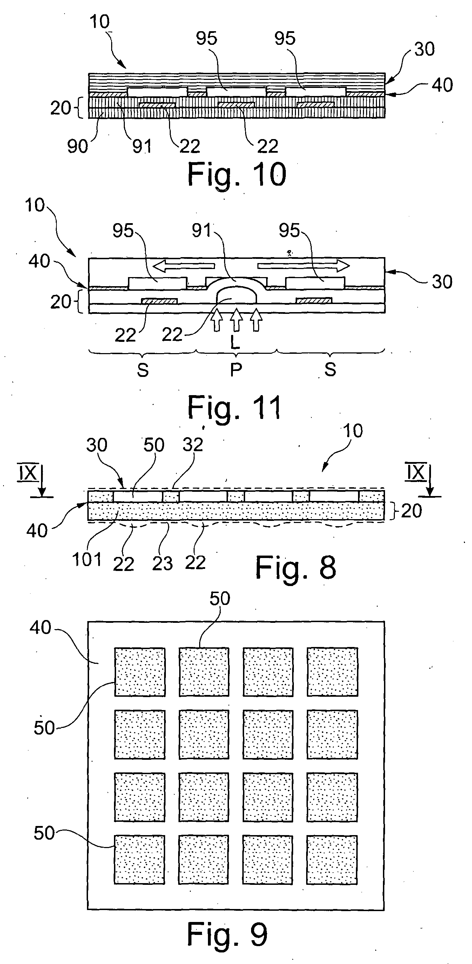

On a illustré sur la figure 8 la possibilité pour la structure proximale 20 de comporter au moins une couche interne, entre les inserts 22 et la structure intercalaire 40. La couche interne 101 peut se déformer pour accompagner le gonflement des inserts 22 et permettre à cette dernière de contacter la structure distale 30, en l'espèce limitée dans cet exemple à une couche de transfert 32. Les inserts 22 sont par exemple logés entre la couche d'interface 23 et la couche interne 101. Les inserts 22 sont répartis comme les ajours 50 de la structure intercalaire 40.

Les propriétés d'absorption et de diffusion du liquide des différentes couches 23 et 101 sont choisies de façon à ce que la migration du liquide puisse s'effectuer de façon préférentielle vers la structure distale 30, là où la structure proximale 20 se superpose à la ou aux zones P de sécrétion du liquide. Autrement dit, la quantité de liquide qui migre latéralement dans la structure proximale 20 est suffisamment faible ou lente pour que les ponts fluidiques entre les structures proximale 20 et distale 30 s'établissent en majorité au-dessus de la ou des zones P de sécrétion du liquide.

Dans un exemple de mise en oeuvre, on utilise pour réaliser la couche d'interface 23, un non-tissé hydrophile Sawabond® 4383 de la société SANDLER, à base de PE/PET, de grammage 30g/m², les inserts 22 sont des grains de polymère superabsorbant FAVOR-PAC® 230, la couche interne 101 est un film 3D perforé commercialisé par la société TREDEGAR sous la référence 40 HEX X26424. La structure intercalaire 40 une mousse hydrophobe de PE de marque Alveolit® et la couche de transfert 32 un non-tissé à base de PET à 45 % et de cellulose à 55 %, et dont la référence est JETEX® 1205C - société ORSA.

Sur les figures 10 et 11, on a représenté un exemple de réalisation dans lequel la structure proximale 20 comporte des première et deuxième couches 90 et 91 ayant un pouvoir drainant vertical, c'est à dire que l'eau migre préférentiellement selon l'épaisseur desdites couches, et entre lesquelles est disposé un matériau hydrogonflant, par exemple sous la forme d'inserts 22 tels que décrits ci-dessus.

La structure intercalaire 40 est formée par une couche d'un adhésif imperméable à l'eau, qui assemble les structures proximale 20 et distale 30. Cette dernière comporte des cavités 95 disposées en regard des inserts 22. La deuxième couche 91 est déformable et peut, lors du gonflement de l'insert 22 sous-jacent, comme illustré à la figure 11, en présence de liquide, contacter le fond de la cavité 95. Ce contact permet le transfert par capillarité ou diffusion du liquide de la structure proximale 20 vers la structure distale 30, le liquide pouvant, une fois dans la structure distale 30, diffuser latéralement pour s'accumuler au sein de celle-ci.

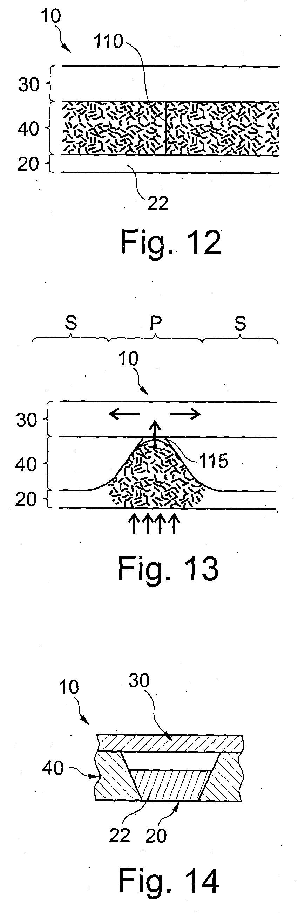

Les figures 12 et 13 visent à illustrer le fait que l'article peut être réalisé de telle sorte qu'après gonflement du matériau hydrogonflant au contact du liquide, un contact direct entre les structures proximale 20 et distale 30 n'ait pas lieu mais qu'un pont fluidique puisse néanmoins s'établir du fait du rapprochement entre les structures proximale 20 et distale 30.

Sur la figure 12, on a représenté l'article avant mise en présence du liquide, et sur la figure 13 après gonflement localisé de la structure proximale 20. On voit sur cet exemple que la structure intercalaire 40 est capable de se déformer pour accompagner l'expansion de la structure proximale 20. A l'état initial, avant mise en présence de l'article avec le liquide, l'épaisseur de la structure intercalaire empêche l'établissement d'un pont fluidique entre les structures proximale et distale. Lorsque la structure intercalaire est localement comprimée par l'expansion de la structure proximale, les structures proximale et distale sont suffisamment rapprochées pour qu'un transfert de liquide de la structure proximale vers la structure distale puisse s'établir par capillarité. Dans cet exemple, la structure intercalaire 40 est par exemple un matériau hydrophobe souple et non absorbant à base de polymères comme par exemple les polyoléfines tel le polyéthylène ou le polyphénylèneéther ou l'acétate de vinyle et d'éthylène, dans lesquels des fentes 110, de préférence en forme de croisillons en vue de dessus, sont ménagées, de telle sorte qu'une fente puisse s'ouvrir lorsque le matériau subit une flexion. Le matériau peut par exemple se présenter sous la forme d'un film souple. Une ouverture 115 se crée alors localement dans la structure intercalaire 40, à travers laquelle le liquide peut s'écouler de la structure proximale vers la structure distale. Dans les zones adjacentes, moins soumises à la flexion, les fentes ne s'ouvrent pas suffisamment pour permettre l'établissement d'un pont fluidique entre les structures proximale et distale, bloquant ainsi le passage du liquide de la structure distale vers la structure proximale.

L'utilisation de tels matériaux permet de réaliser une structure intercalaire non ajourée. Un tel matériau peut aussi selon une autre variante de la présente invention, remplacer l'air dans les cavités de la structure intercalaire entre l'élément gonflant et la structure distale dans les variantes décrites par exemple en référence aux figures 1 à 8. Ces matériaux peuvent aussi être utilisés pour remplir les cavités des figures 10 et 11 et ainsi optimiser le fonctionnement de l'article en éliminant les éventuels risques de retour de liquide vers la plaie, la peau, ou les muqueuses lors de la migration latérale du liquide dans la couche distale. Ceci peut aussi permettre de réaliser des cavités de faible épaisseur dans la couche distale et favoriser l'obtention d'un article mince. Il peut être avantageux que les inserts 22 présentent une forme favorisant leur expansion en direction de la structure distale 30 plutôt que vers la surface sur laquelle est appliqué l'article 10. La figure 14 illustre la possibilité de réaliser la structure proximale 20 sous la forme d'inserts 22 prisonniers des ajours 50 de la structure intercalaire 40, les ajours 50 présentant un rétrécissement de leur section en éloignement de la structure distale 30 et les inserts 22 une forme complémentaire, de telle sorte que mécaniquement l'expansion des inserts vers la surface sur laquelle est posée l'article soit contrariée.

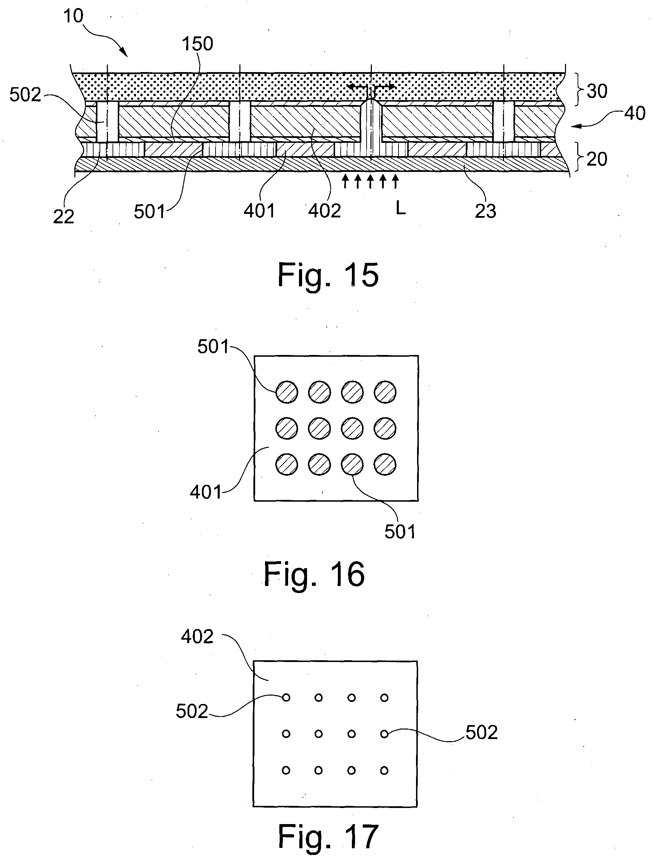

Dans l'ensemble des figures 15 à 17, l'article comporte une structure intercalaire 40 comportant deux couches 401 et 402 superposées l'une sur l'autre, avec éventuellement, comme illustré, interposition d'une couche d'adhésif 150 entre les deux. Les couches 401 et 402 comportent des ajours respectifs 501, 502.

Les ajours 501 de la couche inférieure 401, adjacente à la couche d'interface 23, présentent une section, dans un plan parallèle à la structure proximale 20, supérieure à celles des ajours 502 ménagés dans la couche supérieure 402, cette dernière étant adjacente à la structure distale 30.

Dans les ajours 501 de la couche inférieure 401 sont disposés les inserts 22, comportant un matériau apte à gonfler en présence d'eau. De préférence, les ajours 502 de la couche supérieure 402 ne contiennent pas, initialement, de tels inserts. Ainsi, la couche supérieure 402 a une fonction de maintien de chaque insert 22 dans un ajour 501 de la couche inférieure 401.

Comme cela est représenté sur la figure 15, lorsqu'un insert 22 est sec, il est maintenu entre la couche d'interface 23 et la couche supérieure 402, du fait de la réduction de la section des ajours entre la couche inférieure 401 et la couche supérieure 402. Lorsqu'un insert 22 est placé au contact d'un liquide, le matériau qu'il comporte gonfle progressivement, et s'étend à travers les ajours 502 de la couche supérieure 402, pour atteindre la structure distale 30.

Le procédé de fabrication d'un tel article permet le maintien des inserts 22 de la couche inférieure 401 contre la couche d'interface 23 sans nécessiter l'utilisation d'un adhésif en contact avec les inserts 22, ce qui est avantageux.

Les ajours 501 de la couche inférieure 401 ont de préférence un diamètre (ou une plus grande dimension) compris entre 5 et 25 mm, par exemple 8mm, tandis que les ajours 502 de la couche supérieure 402 ont un diamètre (ou une plus grande dimension) compris entre 1 et 10 mm, par exemple 3 mm.

Dans un exemple de réalisation, chaque couche 401 ou 402 est réalisée en mousse hydrophobe, par exemple de référence TEE 10008. La composition hydrogonflable des inserts 22 est composée des constituants suivants (les pourcentages sont des fractions massiques).

| | **Constituants** | **(en masse) %** |
|---|---|---|
| 1 | Ethanol absolu | 51,903 |
| 2 | PVP K30 (BASF) | 4,152 |
| 3 | Klucel MF pharm (HERCULES) | 1,038 |
| 4 | Favor PAC 230 (EVONIK) | 41,523 |
| 5 | Glycérine 4810 | 1,384 |

Il est avantageux que le matériau apte à gonfler en présence d'eau se présente sous la forme d'une pâte hydrogonflable, comportant, en masse,
- De 10 à 90 % de polymère hydroexpansible, notamment à base de particules de SAP (par exemple Favor PAC 230 de EVONIK),
- 1 à 20% de liants hydrosolubles, par exemple à base de polyvinylpyrrolidone (par exemple Kollidon 30 de BASF ) et/ou d'hydroxypropylcellulose (par exemple Klucel MF Pharm de HERCULES),
- De 0 à 20% de glycérine,
- De 30 à 80 % de liquide d'homogénéisation, par exemple à base d'alcool, par exemple de l'éthanol.

Le fait de disposer d'un matériau hydrogonflable sous la forme d'une pâte permet une manipulation plus aisée lors de la fabrication.

L'invention n'est pas limitée aux exemples décrits et les particularités de réalisation illustrées sur les figures peuvent se combiner au sein de variantes non représentées.

La couche d'interface et/ou toutes les autres couches de l'article peuvent comporter, notamment dans le cas d'un pansement, un ou plusieurs actifs tels que des substances favorisant la cicatrisation ou des substances biocides, des composés hémostatiques ou anti-inflammatoires, ainsi éventuellement qu'un parfum, un agent anti-odeur ou désodorisant. Dans une variante non illustrée, la partie terminale 84 de l'exemple de la figure 7 est reliée par une face à une couche formant réservoir déportée. L'expression « comportant un » doit se comprendre comme étant synonyme de « comportant au moins un » et « compris entre » doit s'entendre bornes incluses, sauf si le contraire est spécifié.

## Revendications

1. Article (10) ayant des propriétés de stockage et/ou d'évacuation d'un liquide (L), notamment pansement, comportant :
- une structure proximale (20) par rapport à une zone d'émission du liquide, perméable au liquide, comportant un matériau capable de gonfler en présence du liquide (L),
- une structure distale (30) par rapport à la zone d'émission du liquide, apte à drainer un liquide (L),
- une structure intercalaire (40) hydrophobe et non absorbante, notamment en un matériau alvéolaire hydrophobe à cellules fermées, s'étendant entre les structures distale (30) et proximale (20), et étant apte d'une part à limiter localement, en l'absence de gonflement dudit matériau, l'échange de liquide entre les structures distale et proximale via le maintien d'un espacement entre ces structures, et d'autre part à autoriser une expansion locale de la structure proximale dans au moins une zone (P) où le gonflement dudit matériau a lieu en réponse à une mise en présence avec un liquide, cette expansion conduisant au rapprochement local de la structure proximale et de la structure distale et au transfert dudit liquide de cette zone de la structure proximale (20) ayant subi l'expansion à la structure distale (30).

2. Article selon la revendication 1, la structure proximale (20) ayant une répartition inhomogène du matériau capable de gonfler, notamment une répartition matricielle selon son plan, en particulier avec un intervalle allant de 1 à 20 mm entre les régions où le matériau capable de gonfler est présent.

3. Article selon l'une quelconque des revendications précédentes, la structure intercalaire (40) étant ajourée, l'expansion de la structure proximale (20) ayant lieu au moins partiellement dans un ajour (50) de la structure intercalaire (40), la répartition des ajours (50) étant notamment sensiblement la même que celle du matériau capable de gonfler.

4. Article selon les revendications 1 ou 2, la structure intercalaire (40) comportant une fente (110) susceptible de s'ouvrir lorsque ladite structure subit une flexion.

5. Article selon l'une quelconque des revendications précédentes, le matériau capable de gonfler est choisi parmi :
- un matériau comportant au moins un polymère superabsorbant (SAP), lui-même choisi parmi les particules de polymères superabsorbants ou les non tissés à base de fibres de cellulose et de particules d'un polymère superabsorbant
- un matériau alvéolaire hydrogonflant, notamment une mousse de polyuréthane hydrophile (PU).

6. Article selon l'une quelconque des revendications précédentes, la structure proximale comportant une couche d'interface (23) destinée à venir en contact avec la zone d'émission du liquide, notamment une couche d'interface au contact d'inserts (22) du matériau capable de gonfler et au contact de la structure intercalaire (40), la couche d'interface (23) étant notamment choisie parmi les matériaux textiles non absorbants, de préférence parmi les non tissés hydrophobes.

7. Article selon l'une quelconque des revendications précédentes, la structure intercalaire (40) assurant la fixation de la structure proximale (20) sur la structure distale (30), notamment comportant une couche d'adhésif imperméable à l'eau, s'étendant de façon discontinue entre la structure distale et la structure proximale, de façon à ménager au moins un ajour (50) où le gonflement du matériau de la structure proximale conduit à un transfert du liquide vers la structure distale par contact entre la structure proximale et la structure distale.

8. Article selon l'une quelconque des revendications précédentes, la structure proximale (20) étant entièrement située hors de la structure intercalaire (40) en l'absence de gonflement du matériau apte à gonfler.

9. Article selon l'une quelconque des revendications précédentes, la structure proximale (20) étant engagée dans la structure intercalaire (40) en l'absence de gonflement du matériau apte à gonfler, et s'étendant sur une partie seulement de l'épaisseur de celle-ci, ou la structure proximale (20) comportant ou étant constituée par des inserts (22) du matériau apte à gonfler, notamment portés par une couche d'interface (23), la structure intercalaire (40) comportant de préférence des ajours (50) dans lesquels sont engagés au moins partiellement les inserts (22), la structure intercalaire (40) ayant une épaisseur suffisante pour qu'avant gonflement les inserts ne contactent pas la structure distale, la structure intercalaire ayant notamment une épaisseur supérieure à celle des inserts, et suffisamment faible pour que le gonflement des inserts (22) les amène à contacter la structure distale (30).

10. Article selon l'une quelconque des revendications précédentes, la structure distale (30) comportant au moins une cavité (95) débouchant en direction de la structure proximale (20), cette cavité (95) se superposant au moins partiellement à une zone de la structure proximale où le gonflement est susceptible d'avoir lieu, et de préférence se superposant au moins partiellement à un ajour (50) de la structure intercalaire (40).

11. Article selon l'une quelconque des revendications précédentes, la structure intercalaire (40) comportant deux couches (401,402) ajourées, dont une couche inférieure (401) du côté de la structure proximale (20) et une couche supérieure (402) du côté de la structure distale (30), les ajours (502) de la couche supérieure (402) ayant une section moindre que les ajours (501) de la couche inférieure (401), de préférence ayant une plus grande dimension, notamment un diamètre, compris entre 5 et 25 mm et les ajours (501) de la couche supérieure (402) ayant une plus grande dimension, notamment un diamètre, compris entre 1 et 10 mm, et se superposant à ces derniers, le matériau capable de gonfler étant contenu au moins partiellement, lorsque sec, dans les ajours (501) de la couche inférieure (401), mieux remplissant en totalité les ajours (501) de la couche inférieure (401).

12. Article selon l'une quelconque des revendications précédentes, le matériau capable de gonfler étant pâteux, au moins lors de la confection de l'article, notamment lors de son introduction dans des ajours (50 ; 501) de la structure intercalaire, par exemple comportant un polymère hydroexpansible, notamment à base de particules de SAP et un liant hydrosoluble, notamment à base de polyvinylpyrrolidone et/ou d'hydroxypropylcellulose, par exemple le matériau capable de gonfler ayant la formulation suivante, en masse par rapport à la masse totale du mélange :
- de 10 à 90% de polymère(s) hydroexpansible(s), notamment à base de particules de SAP,
- 1 à 20% de liant(s) hydrosoluble(s), par exemple à base de polyvinylpyrrolidone et/ou d'hydroxypropylcellulose,
- de 0 à 20% de glycérine, et
- de 30 à 80% de liquide d'homogénéisation, notamment à base d'alcool, de préférence de l'éthanol.

13. Article selon l'une quelconque des revendications précédentes, la structure distale (30) comportant une pluralité de cavités (95) disposées selon une répartition matricielle, les cavités étant de préférence réparties comme les zones de la structure proximale susceptibles de gonfler.

14. Article selon l'une quelconque des revendications précédentes, la structure distale (30) comportant une couche formant réservoir (31), ayant une capacité d'absorption de liquide supérieure ou égale à 500 g/m².

15. Article selon l'une quelconque des revendications précédentes, la structure distale (30) est formée d'une couche de transfert et est dépourvue de couche formant réservoir ayant une capacité d'absorption de liquide supérieure ou égale à 800 g/m², ou l'article comportant une couche déportée formant réservoir (80) décalée latéralement par rapport à la structure intercalaire, et la structure distale comportant une couche de transfert (32) reliée à la couche formant réservoir déportée (80), ou l'article comportant une couche de transfert (32) présentant une partie terminale libre (84) de laquelle le liquide peut s'évaporer, la couche de transfert (32) se superposant partiellement à la structure intercalaire.

## Patentansprüche

1. Artikel (10) mit Speicher- und/oder Aufsaugeigenschaften für eine Flüssigkeit (L), insbesondere Verband, mit:
- einer in Bezug auf eine Emissionszone für die Flüssigkeit proximale, für die Flüssigkeit durchlässige Struktur (20), die ein Material aufweist, das in der Lage ist, in Gegenwart der Flüssigkeit (L) zu quellen,
- einer in Bezug auf die Emissionszone für die Flüssigkeit distalen Struktur (30), die in der Lage ist, eine Flüssigkeit (L) abzuleiten,
- einer hydrophoben und nicht absorbierenden Zwischenstruktur (40), insbesondere aus einem hydrophoben zellenförmigen Material mit geschlossenen Zellen, die sich zwischen der distalen Struktur (30) und der proximalen Struktur (20) erstreckt und in der Lage ist, einerseits, in Abwesenheit der Quellung des genannten Materials, den Austausch von Flüssigkeit zwischen den distalen und proximalen Strukturen lokal zu begrenzen durch Aufrechterhaltung eines Abstands zwischen diesen Strukturen, und andererseits eine lokale Expansion der proximalen Struktur in wenigstens einer Zone (P), wo die Quellung des Materials als Reaktion auf das Zusammenführen mit einer Flüssigkeit stattfindet, zuzulassen, wobei diese Expansion zur lokalen Annäherung der proximalen Struktur und der distalen Struktur und zur Übertragung der Flüssigkeit aus dieser Zone der proximalen Struktur (20), die der Expansion unterlag, auf die distale Struktur (30) führt.

2. Artikel nach Anspruch 1, bei dem die proximale Struktur (20) eine inhomogene Verteilung des quellfähigen Materials aufweist, insbesondere eine matrixförmige Verteilung in ihrer Ebene, insbesondere mit einem Intervall von 1 bis 20 mm zwischen den Regionen, in denen das quellfähige Material vorhanden ist.

3. Artikel nach einem der vorstehenden Ansprüche, bei dem die Zwischenstruktur (40) durchbrochen ist, wobei die Expansion der proximalen Struktur (20) wenigstens zum Teil in einem Durchbruch (50) der Zwischenstruktur (40) stattfindet und wobei die Verteilung der Durchbrüche (50) insbesondere im wesentlichen die gleiche ist wie diejenige des quellfähigen Materials.

4. Artikel nach Anspruch 1 oder 2, bei dem die Zwischenstruktur (40) einen Schlitz (110) aufweist, der in der Lage ist, sich zu öffnen, wenn diese Struktur einer Biegung unterliegt.

5. Artikel nach einem der vorstehenden Ansprüche, bei dem das quellfähige Material ausgewählt ist unter:
- einem Material, das wenigstens ein superabsorbierendes Polymer (SAP) aufweist, das seinerseits ausgewählt ist unter superabsorbierenden Polymerpartikeln oder Vliesen auf der Basis von Zellulosefasern und superabsorbierenden Polymerpartikeln,
- einem in Wasser quellenden zellenförmigen Material, insbesondere einem Schaum aus hydrophilem Polyurethan (PU).

6. Artikel nach einem der vorstehenden Ansprüche, bei dem die proximale Struktur eine Grenzschicht (23) aufweist, die dazu bestimmt ist, mit der Emissionszone für die Flüssigkeit in Kontakt zu kommen, insbesondere eine Grenzschicht in Kontakt mit Einlagen (22) des quellfähigen Materials und in Kontakt mit der Zwischenstruktur (40), wobei die Zwischenschicht (23) insbesondere ausgewählt ist unter nicht absorbierenden textilen Materialien, vorzugsweise unter hydrophoben Vliesen.

7. Artikel nach einem der vorstehenden Ansprüche, bei dem die Zwischenstruktur (40) die Befestigung der proximalen Struktur (20) an der distalen Struktur (30) sicherstellt, insbesondere mit einer Klebeschicht, die für Wasser undurchlässig ist und sich diskontinuierlich zwischen der distalen Struktur und der proximalen Struktur erstreckt, derart, dass wenigstens ein Durchbruch (50) gebildet wird, wo das Quellen des Materials der proximalen Struktur zu einer Übertragung der Flüssigkeit zu der distalen Struktur durch Kontakt der proximalen Struktur mit der distalen Struktur führt.

8. Artikel nach einem der vorstehenden Ansprüche, bei dem die proximale Struktur (20) ohne Quellung des quellfähigen Materials vollständig außerhalb der Zwischenstruktur (40) liegt.

9. Artikel nach einem der vorstehenden Ansprüche, bei dem die proximale Struktur (20) ohne Quellung des quellfähigen Materials mit der Zwischenstruktur (40) in Eingriff ist und sich nur auf einem Teil der Dicke derselben erstreckt, oder die proximale Struktur (20) Einlagen (22) aus dem quellfähigen Material aufweist oder durch diese gebildet wird, die insbesondere durch eine Zwischenschicht (23) gehalten sind, wobei die Zwischenstruktur (40) vorzugsweise Durchbrüche (50) aufweist, in welche die Einlagen (22) zumindest zum Teil eingreifen, wobei die Zwischenstruktur (40) eine so große Dicke aufweist, dass die Einlagen vor dem Quellen nicht die distale Struktur berühren, wobei die Zwischenstruktur insbesondere eine Dicke hat, die größer ist als diejenige der Einlagen, und schwach genug ist, dass das Quellen der Einlagen (22) sie dazu bringt, die distale Struktur (30) zu berühren.

10. Artikel nach einem der vorstehenden Ansprüche, bei dem die distale Struktur (30) wenigstens eine Höhlung (95) aufweist, die sich in Richtung auf die proximale Struktur (20) öffnet, wobei diese Höhlung (95) zumindest zum Teil mit einer Zone der proximalen Struktur überlappt, wo die Quellung stattfinden kann, und vorzugsweise zumindest zum Teil mit einem Durchbruch (50) der Zwischenstruktur (40) überlappt.

11. Artikel nach einem der vorstehenden Ansprüche, bei dem die Zwischenstruktur (40) zwei durchbrochene Schichten (401, 402) aufweist, von denen eine untere Schicht (401) auf der Seite der proximalen Struktur (20) und eine obere Schicht (402) auf der Seite der distalen Struktur (30) liegt, wobei die Durchbrüche (502) der oberen Schicht (402) einen Querschnitt haben, der kleiner ist als die Durchbrüche (501) der unteren Schicht (401), die vorzugsweise eine größere Abmessung haben, insbesondere einen Durchmesser, zwischen 5 und 25 mm, und die Durchbrüche (501) der oberen Schicht (402) eine größere Abmessung haben, insbesondere einen Durchmesser, zwischen 1 und 10 mm, und sich mit diesen letzteren überlagern, wobei das quellfähige Material, wenn es trocken ist, zumindest zum Teil in den Durchbrüchen (501) der unteren Schicht (401) enthalten ist und am besten die Durchbrüche (501) der unteren Schicht (401) vollständig ausfüllt.

12. Artikel nach einem der vorstehenden Ansprüche, bei dem das quellfähige Material zumindest bei der Herstellung des Artikels, insbesondere bei seinem Einführen in die Durchbrüche (50; 501) der Zwischenstruktur pastös ist, beispielsweise ein in Wasser expandierbares Polymer aufweist, insbesondere auf der Basis von Partikeln aus SAP und einem wasserlöslichen Bindemittel, insbesondere auf der Basis von Polyvinylpyrrolidon und/oder Hydroxypropylzellulose, wobei das quellfähige Material beispielsweise die folgende Formulierung hat, angegeben in Masse relativ zu der gesamten Masse des Gemisches:
- 10 oder 90% hydroexpandierbare(s) Polymer(e), insbesondere auf der Basis von Partikeln aus SAP,
- 1 bis 20% wasserlösliche(s) Bindemittel, beispielsweise auf der Basis von Polyvinylpyrrolidon und/oder Hydroxypropylzellulose,
- 0 bis 20% Glyzerin, und
- 30 bis 80% Homogenisierungsflüssigkeit, insbesondere auf der Basis von Alkohol, vorzugsweise Ethanol.

13. Artikel nach einem der vorstehenden Ansprüche, bei dem die distale Struktur (30) mehrere Höhlungen (95) aufweist, die gemäß einer Matrixverteilung angeordnet sind, wobei die Höhlungen vorzugsweise so verteilt sind wie die quellfähigen Zonen der proximalen Struktur.

14. Artikel nach einem der vorstehenden Ansprüche, bei dem die distale Struktur (30) eine Speicherschicht (31) aufweist, die eine Aufnahmekapazität für Flüssigkeit von 500 g/m² oder mehr hat.

15. Artikel nach einem der vorstehenden Ansprüche, bei dem die distale Struktur (30) durch eine Übertragungsschicht gebildet wird und keine Speicherschicht mit einer Aufnahmekapazität für Flüssigkeit von 800 g/m² oder mehr aufweist oder der Artikel eine deportierte Schicht aufweist, die einen Speicher (80) bildet, der gegenüber der Zwischenstruktur seitlich versetzt ist, und die distale Struktur eine Übertragungsschicht (32) aufweist, die mit der deportierten, den Speicher (80) bildenden Schicht in Verbindung steht, oder der Artikel eine Übertragungsschicht (32) aufweist, die einen freien Endabschnitt (84) bildet, von dem die Flüssigkeit verdampfen kann, wobei die Übertragungsschicht (32) die Zwischenschicht partiell überlagert.

## Claims

1. An item (10) having liquid (L) storage and/or discharge properties, in particular a dressing, comprising:
- a liquid-permeable proximal structure (20) which is proximal with respect to a liquid emission zone, comprising a material capable of swelling in the presence of the liquid (L),
- a distal structure (30) which is distal with respect to the liquid emission zone, capable of draining a liquid (L),
- a hydrophobic and nonabsorbent insert structure (40), in particular in a closed-cell hydrophobic alveolar material, extending between the distal (30) and proximal (20) structures, and being capable firstly of locally limiting, when said material is not swollen, the exchange of liquid between the distal and proximal structures by maintaining a gap between these structures, and secondly of allowing a local expansion of the proximal structure in at least one zone (P) where said material is swollen in response to coming into contact with a liquid, this expansion causing the proximal structure and the distal structure to move closer together locally and the transfer of said liquid from this zone of the proximal structure (20) having undergone expansion to the distal structure (30).

2. The item as claimed in claim 1, the proximal structure (20) having a nonuniform distribution of the material capable of swelling, in particular a matrix distribution according to its plane, in particular with a gap ranging from 1 to 20 mm between the regions where the material capable of swelling is present.

3. The item as claimed in either one of the preceding claims, the insert structure (40) having openings, the expansion of the proximal structure (20) at least partially taking place in an opening (50) of the insert structure (40), in particular the distribution of the openings (50) being substantially the same as that of the material capable of swelling.

4. The item as claimed in claim 1 or 2, the insert structure (40) comprising a slot (110) capable of opening up when said structure undergoes bending.

5. The item as claimed in any one of the preceding claims, the material capable of swelling being chosen from:
- a material comprising at least one superabsorbent polymer (SAP), itself chosen from particles of superabsorbent polymers or nonwovens based on cellulose fibers and on particles of a superabsorbent polymer,
- a water-swelling alveolar material, in particular a hydrophilic polyurethane (PU) foam.

6. The item as claimed in any one of the preceding claims, the proximal structure comprising an interface layer (23) intended to come into contact with the liquid emission zone, in particular an interface layer in contact with inserts (22) of the material capable of swelling and in contact with the insert structure (40), in particular the interface layer (23) being chosen from nonabsorbent textile materials, preferably from hydrophobic nonwovens.

7. The item as claimed in any one of the preceding claims, the insert structure (40) providing the attachment of the proximal structure (20) to the distal structure (30), in particular comprising a water-impermeable adhesive layer extending discontinuously between the distal structure and the proximal structure, so as to make at least one opening (50) where the swelling of the material of the proximal structure results in a transfer of the liquid to the distal structure by contact between the proximal structure and the distal structure.

8. The item as claimed in any one of the preceding claims, the proximal structure (20) being entirely located outside the insert structure (40) in the absence of swelling of the material capable of swelling.

9. The item as claimed in any one of preceding claims, the proximal structure (20) being inserted in the insert structure (40) in the absence of swelling of the material capable of swelling, and extending over only a part of the thickness thereof, or the proximal structure (20) comprising or being made up of inserts (22) of the material capable of swelling, in particular being borne by an interface layer (23), the insert structure (40) preferably comprising openings (50) in which the inserts (22) are at least partially inserted, the insert structure (40) having a sufficient thickness so that, before swelling, the inserts do not come into contact with the distal structure, the insert structure having in particular a thickness greater than that of the inserts, and sufficiently small for the swelling of the inserts (22) to bring them into contact with the distal structure (30).

10. The item as claimed in any one of the preceding claims, the distal structure (30) comprising at least one cavity (95) which opens out in the direction of the proximal structure (20), this cavity (95) being at least partially superimposed on a zone of the proximal structure where the swelling is liable to take place, and preferably being at least partially superimposed on an opening (50) of the insert structure (40).

11. The item as claimed in any one of the preceding claims, the insert structure (40) comprising two layers (401, 402) with openings, including one lower layer (401) on the side of the proximal structure (20) and one upper layer (402) on the side of the distal structure (30), the openings (502) of the upper layer (402) having a smaller cross section than the openings (501) of the lower layer (401), in particular the openings (501) of the lower layer (401) having a larger dimension, in particular a diameter, of between 5 and 25 mm and the openings (501) of the upper layer (402) having a larger dimension, in particular a diameter, of between 1 and 10 mm, and being superimposed on the latter, the material capable of swelling being at least partially contained, when dry, in the openings (501) of the lower layer (401), better totally filling the openings (501) of the lower layer (401).

12. The item as claimed in any one of the preceding claims, the material capable of swelling being pasty, at least during the preparation of the item, in particular during its introduction into openings (50; 501) of the insert structure, for example comprising a water-expandable polymer, in particular based on SAP particles, and a water-soluble binder, in particular based on polyvinylpyrrolidone and/or on hydroxypropylcellulose, for example the material capable of swelling having the following formulation, by weight relative to the total weight of the mixture:
from 10% to 90% of water-expandable polymer(s), in particular based on SAP particles, from 1% to 20% of water-soluble binder(s), for example based on polyvinylpyrrolidone and/or of hydroxypropylcellulose,
from 0% to 20% of glycerol, and
from 30% to 80% of homogenization liquid, in particular based on alcohol, preferably ethanol.

13. The item as claimed in any one of the preceding claims, the distal structure (30) comprising a plurality of cavities (95) arranged according to a matrix distribution, the cavities preferably being distributed like the zones of the proximal structure which are capable of swelling.

14. The item as claimed in any one of the preceding claims, the distal structure (30) comprising a layer forming a reservoir (31), having a liquid absorption capacity greater than or equal to 500 g/m².

15. The item as claimed in any one of claims 1 to 24, the distal structure (30) being formed from a transfer layer and being devoid of a layer forming a reservoir having a liquid absorption capacity greater than or equal to 800 g/m², or the item comprising an offset layer forming a reservoir (80) which is laterally offset with respect to the insert structure, and the distal structure comprising a transfer layer (32) connected to the offset layer forming a reservoir (80), or the item comprising a transfer layer (32) having a free end part (84) from which the liquid may evaporate, the transfer layer (32) being partially superimposed on the insert structure.
